# EUROPEAN PATENT APPLICATION

(11) **EP 4 736 773 A1**
(43) Date of publication of application: **06.05.2026**
(21) Application number: 24209549.5
(22) Date of filing: 29.10.2024
(51) Int. Cl.: A61B 6/50, G06T 7/00, G06T 7/11

(54) **SYSTEM AND METHOD FOR DETERMINING INNER AND OUTER VESSEL CURVE**

(71) Applicant: Koninklijke Philips N.V., 5656 AG Eindhoven (NL)
(72) Inventor: THAYYULLATHIL,, Hemanth, Eindhoven (NL); RABOTNIKOV, Mark, Eindhoven (NL); SETTY, Vasavi, 5656AG Eindhoven (NL)
(74) Representative: Philips Intellectual Property & Standards

(57) **Abstract**

A method and system for preprocedural planning is disclosed. The method includes receiving a 3D medical image (205) comprising 3D segmentation (210) of a vessel (220). The 3D segmentation (210) comprises a centerline (214A) of the vessel (220) and contours (218 A-N) of an inner wall of the vessel (220). The centerline (214A) and contours (218 A-N) of the inner wall of the vessel (220) are represented as centerline points (222 A-N) and contour points (224 A-N) in a 3D space. The method includes identifying an inner (212) and an outer curve (216) of the inner wall of the vessel (220) and outputting the inner (212) and the outer curve (216) of inner wall of vessel (220) on a display. Additionally, when an aneurysm is detected, the method (100) includes recomputing the centerline (214B), modified contours in the region of aneurysm and inner (212) and outer (216) curves.

## Description

### FIELD OF THE INVENTION

The invention relates to image processing and more particularly relates to techniques for representing the segmentation of features derived from 3D contours in a medical image.

### BACKGROUND OF THE INVENTION

CT angiography (CTA) is commonly used as a preprocedural imaging tool in various medical contexts, especially for vascular and cardiac procedures. This feature aids the clinicians in preprocedural planning for Endovascular stent-graft placement. The primary purpose of this feature is to aid with an estimation of the stent length to be placed in a vessel. Additionally, the length measurement of the stent could be used to confirm suspected abnormality. Endovascular stents or stent grafts have become a viable treatment for thoracic and abdominal aortic aneurysms in both elective and emergency situations.

The ability of stent deployment in a vessel is largely dependent on the compatibility between vessel geometry and the stent hardware. The deployment of the stent in a blood vessel is complex and involves several critical challenges. Such challenges for deployment of the stent in the blood vessel include navigating the stent through tortuous anatomy, managing varying plaque characteristics of the vessels, minimizing the risk of dissection or perforation, and ensuring precise positioning of the stent to cover the entire lesion while avoiding placement over critical branch vessels.

One of such critical challenges of stent deployment involves manoeuvring the stent through winding or narrow vessels, especially in patients with complex vascular structures. Another challenge involves improper positioning of the stent in the vessel that can damage the vessel wall during deployment, leading to complications such as dissection or perforation. The complex geometry of vessels, along with patient-specific factors such as vessel size, blood flow dynamics, and existing comorbidities, can influence the complexity and outcome of the procedure. These factors also increase the risk of the stent recoiling after deployment or migrating from its intended location, which could result in inadequate coverage or re-narrowing of the vessel. Wrong stent deployment may lead to complications, such as stent graft migration, endo leaks, blood clot formations, vessel injury as well various graft complications.

### SUMMARY OF THE INVENTION

It is an object of the invention to provide improved preprocedural planning and/or computer aided diagnosis methods, systems and computer program products by aiding clinicians in preprocedural planning for endovascular stent-graft placement and/or computer aided diagnosis. Additionally, the accurate length measurement of the stent may be used to confirm a precise suspected abnormality.

The ability of placement of stent-graft is largely dependent on the compatibility between aneurysm geometry and the stent hardware. The inner and outer curves of a vessel play a primary role in establishing this compatibility. The placement of a stent-graft is efficient only when the measurements of the vessel, such as its length, luminal diameter, and collapsed diameter are estimated accurately. These measurements are most precise when they are based on both the inner and outer curves of the vessel. This is because, during stent deployment, the guidewire in a long aneurysm neck is forced to follow the path of the aneurysm neck, navigating along the inner and outer curves.

Starting from preprocedural planning through to stent deployment, the inner and outer curves of the vessel play a crucial role. The inner and outer curve lengths of the vessel more accurately reflect the length of the deployed endograft and are more precise than centerlines of the vessel in planning thoracic endografts. The greater the curvature and the larger the vessel, the more significant the underestimation when centerlines are used for length calculation. The concept of using outer and inner curves, especially for the aorta, shows promising results, even accounting for longitudinal strain and axial twists.

While centerlines are useful tools in vessel analysis, incorporating the inner and outer curves adds significant value. When both features and measurements are used together in vessel analysis, diagnosis, and preprocedural planning, they can certainly lead to new and improved applications in radiology.

To this end the invention provides a system and a computer-implemented method for preprocedural planning and/or computer aided diagnosis. The method comprises the steps of receiving a 3D medical image comprising a 3D segmentation of a vessel. The 3D segmentation comprises a centerline of the vessel and a plurality of contours of an inner wall of the vessel. The centerline of the vessel and the plurality of contours of the inner wall of the vessel are represented as centerline points and contour points in a 3D space. The method comprises the steps of identifying an inner curve and an outer curve of the inner wall of the vessel in the 3D segmentation of the vessel, using a plurality of direction vectors orthogonal from the plurality of centerline points to the plurality of corresponding contour points. The method comprises the steps of outputting the inner curve and the outer curve of the inner wall of the vessel on a display together with the 3D medical image and the 3D segmentation of the vessel.

It is an advantage of the invention to provide for an accurate estimation of the stent length to be placed, especially for curved regions of the aorta.

These and other aspects of the invention are apparent from and will be elucidated with reference to the embodiments described hereinafter.

In an embodiment of the invention, using the plurality of direction vectors orthogonal from the plurality of centerline points to the plurality of corresponding contour points comprises the step of mapping the plurality of centerline points and the plurality of contour points into virtual latitudes and longitudes. Virtual latitudes and longitudes are coordinates in the form of angles. This provides an effective and intuitive step for establishing correspondence between contour points across cross-sectional contours along a vessel. Ensuring accurate correspondence between plurality of contour points is essential for making meaningful length measurements along the vessel's long axis. This becomes even more critical in the presence of twists, which are frequently encountered in diseased blood vessels.

In an embodiment of the invention, the computer-implemented method further comprising the step of identifying, a region of the vessel in the 3D medical image that comprises an aneurysm. It further comprises the step of recomputing, the centerline in the identified region for obtaining a modified centerline. Furthermore, the method comprises the step of generating the modified centerline that replaces the centerline. This approach provides a straightforward and essential step to obtain the contours of the lumen, preventing misleading measurements caused by the aneurysm walls, which could otherwise lead to inaccurate length calculations. Additionally, this solution enables visualization of the centerline and cross-sectional contours, accurately representing the lumen prior to aneurysm expansion.

In a further embodiment, the step for recomputing the centerline in the identified region comprises (i) interpolating using two points each on either side of the identified region and (ii) resampling the interpolated points to keep them equidistant, such as to identify the modified centerline in the identified region. In particular, interpolating using a polynomial (function) is applied. The polynomial has a certain degree and in particular, interpolating using a cubic polynomial (third degree) is advantageous. Recomputing the centerline in this manner offers the advantage that the interpolated centerline closely follows the geometry of the lumen's central points, even in cases with bends.

In further embodiment of the invention, the computer-implemented method comprises the step of determining the plurality of contours between the contour points at the start and at the end of the identified region in the 3D medical image. It further comprises the step of computing a modified radii of the plurality of contours in the identified region of the 3D medical image by interpolating a radii of the contour points of the plurality of contours along the entire length between the start and the end of the identified region in the 3D medical image. It further comprises the step of generating modified contours from the modified radii and the modified centerline. This method offers the advantage of providing contours that closely match the natural lumen, even in the presence of any aneurysm, and performs well across blood vessels of varied shapes and sizes.

In a further embodiment of the invention, the inner curve and the outer curve are computed from the modified centerline and the modified contour. An advantage of this embodiment is that it assists clinicians by providing more accurate measurements for stent graft design.
It will be appreciated by those skilled in the art that two or more of the above-mentioned options, implementations, and/or aspects of the invention may be combined in any way deemed useful.

### BRIEF DESCRIPTION OF THE DRAWINGS

These and other aspects of the system and the method according to the invention will be further elucidated and described with reference to the drawing, in which:
Fig. 1 is a schematic representation of a method according to an embodiment of the present invention.
Fig. 2 illustrates a representation of a 3D medical image of a vessel and a schematic of 3D segmentation of the vessel according to an embodiment of the present invention.
Fig. 3 is a representation of the 3D medical image illustrating the vessel and showing an original centerline and a modified centerline in the region of an aneurysm, with the chosen start and end points of the aneurysm as indicated according to an embodiment of the present invention.
Fig. 4 is a representation of an identified inner curve and an outer curve according to an embodiment of the present invention.
Fig. 5 is an illustration of a schematic of a 3D segmentation of the vessel showing a correspondence between the contour points according to an embodiment of the present invention.
Fig 6 is a schematic representation of a system according to an embodiment of the present invention.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

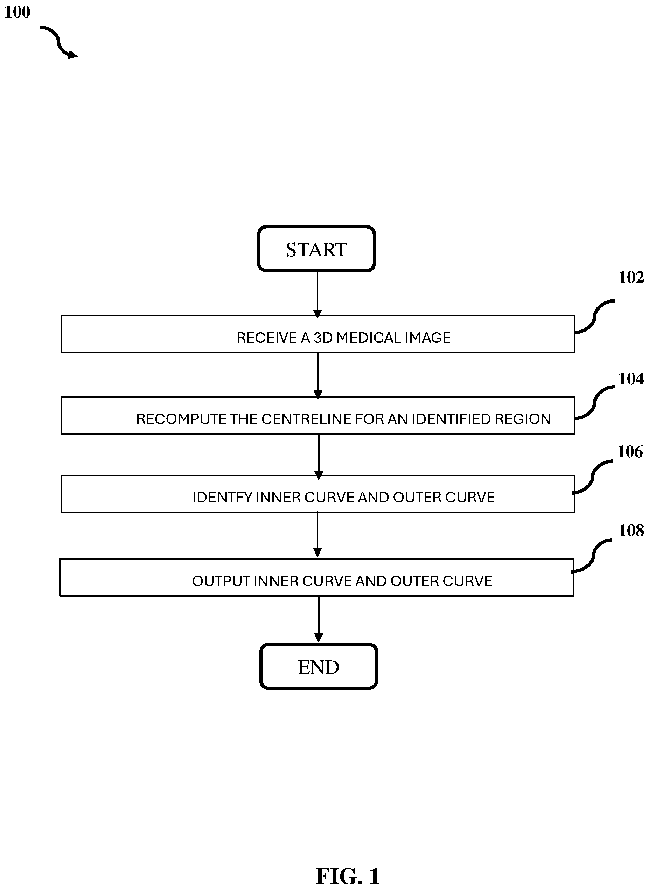

Fig. 1 shows a flowchart of an embodiment of a method 100 for preprocedural planning according to the invention. The objective of the method 100 is to identify the inner and outer curves of a vessel, an aorta, for example. Additionally, in case of aneurysm, the method 100 recomputes a centerline and contours in the region of the aneurysm.

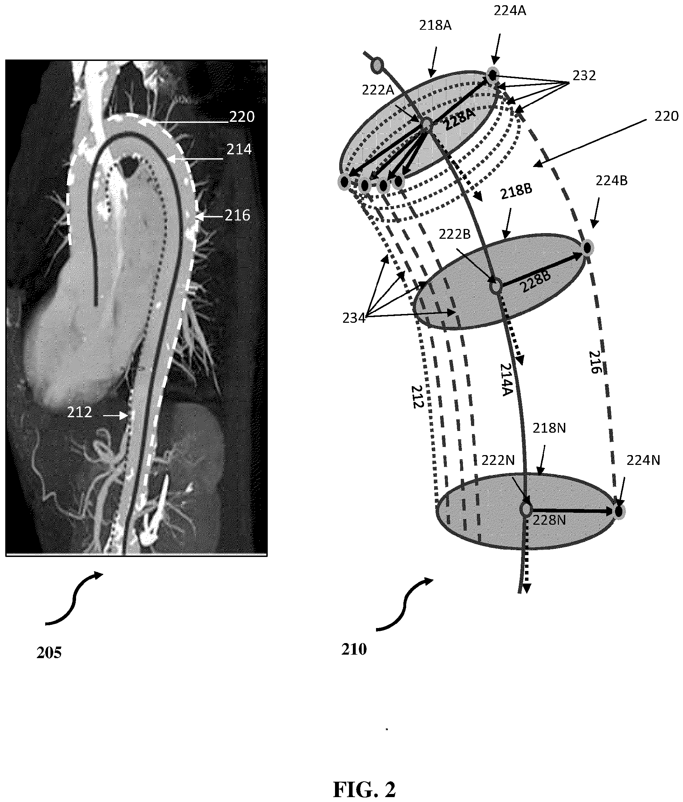

The process of method 100 starts at the initialization step. The method as shown in Fig. 1 is explained in conjunction with Fig. 2, Fig. 3 and Fig. 4. Fig. 2 illustrates a representation of a 3D medical image 205 comprising a vessel 220 and a schematic of 3D segmentation 210 of the vessel 220, according to an embodiment of the present invention.

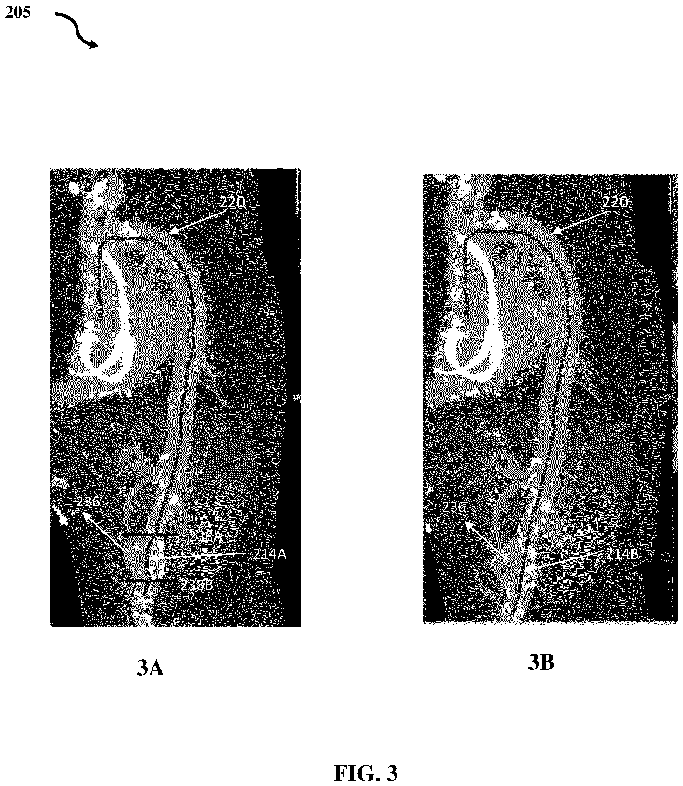

It is to be noted that, in one example, the schematic of 3D segmentation 210 of the vessel 220 as shown in Fig. 2 may be a part of a vessel 220 or may be an entire vessel. In another example, the schematic of 3D segmentation 210 of vessel 220 as shown in Fig. 2 may also be an identified region 236 of the vessel 220 in the 3D medical image 205 that comprises an aneurysm 236 (as shown in Fig. 3). Fig. 3 is a representation of the 3D medical image 205 illustrating the vessel 220 with an original centerline (as shown in 3A) and a modified centerline (as shown in 3B) in the region of the aneurysm, with the chosen start (238A) and end points (238B) of the aneurysm as indicated according to an embodiment of the present invention. It should be noted that the identified region 236 may represent any type of anomaly in the vessel where endpoints can be detected and is not limited solely to aneurysms as mentioned herein. Each step of the method 100 are explained in detail below.

At step 102, the method 100 includes receiving the 3D medical image 205. The 3D medical image 205 includes the 3D segmentation 210 of the vessel 220. In one example, the vessel 220 may be a blood vessel.

The schematic of 3D segmentation 210 of the vessel 220 comprises a centerline 214A of the vessel 220 and a plurality of contours 218 A-N of an inner wall of the vessel 220. For conciseness, the vessel 220 is segmented using the centerline 214A as a reference to generate the schematic of 3D segmentation 210 of the vessel. The centerline 214A of the vessel 220 and the plurality of contours 218 A-N of the inner wall of the vessel 220 are represented as centerline points 222 A-N and contour points 224 A-N in a 3D space.

Referring to the 3D medical image 205, it is noted that both the inner and outer curve lines are positioned along the surface of the inner wall of the vessel 220. In the schematic representation of 3D segmentation 210 of the vessel 220, the inner wall is defined by the surface formed by contour points 224 A-N. These points are part of a plurality of contours 218 A-N, which are distributed around each centerline point 222 A-N.

In one embodiment, the schematic of 3D segmentation 210 as shown in Fig. 2 is segmented using the centerline 214A of the vessel 220 as the reference and the inner wall is further mapped into latitudes 232 and longitudes 234, resulting from a stack of 3D contours 218 A-N.

Also, it's important to note that, the method 100 includes a step 104 for recomputing the centerlines 214B and the vessel inner contours 218 A-N in the region of aneurysms (if any), to identify accurate inner curve 212 and outer curves 216. The steps for recomputing the centerlines 214B and the vessel inner contours 218 A-N in the region of aneurysms (if any) as shown in Fig. 3 is described in detail below.

At step 104, the method 100 includes recomputing the centerline 214B in the identified region 236 (as shown in Fig. 3). It is to be noted that the original centerline is shown by reference numeral 214A, with the chosen start 238A and end points 238B of the aneurysm 236 as indicated and the modified centerline is shown by reference numeral 214B in the region of the aneurysm 236. The recomputed centerline 214B is then used to compute the modified contours in the identified region 236.

For recomputing the centerline 214A, the method 100 includes identifying the region 236 of the vessel 220 in the 3D medical image 205 that comprises an aneurysm 236. The steps for recomputing the centerline 214A in the identified region 236 comprise (i) interpolating with a polynomial using two points each on either side of the identified region 236 and (ii) resampling the interpolated points to keep them equidistant, such as to identify the modified centerline 214B in the identified region 236 and generating the modified centerline 214B that replaces the original centerline 214A. In particular, interpolating using a polynomial (function) is applied. The polynomial has a certain degree and in particular, interpolating using a cubic polynomial (third degree) is advantageous. Recomputing the centerline in this manner offers the advantage that the interpolated centerline closely follows the geometry of the lumen's central points, even in cases with bends. It should be noted that this step is not limited to only cubic interpolation and may also include linear or quadratic polynomial interpolation. The method 100 includes a step of determining the plurality of contours 218 A-N between the contour points 224 A-N at the start 238A and at the end 238B of the identified region 236 in the 3D medical image 205, computing a modified radii of the plurality of contours 218 A-N in the identified region 236 of the 3D medical image 205 by interpolating a radii of the contour points 224 A-N of the plurality of contours 218 A-N along the entire length between the start 238A and the end 238B of the identified region 236 in the 3D medical image 205 and generating modified contours from the modified radii and the modified centerline 214B.

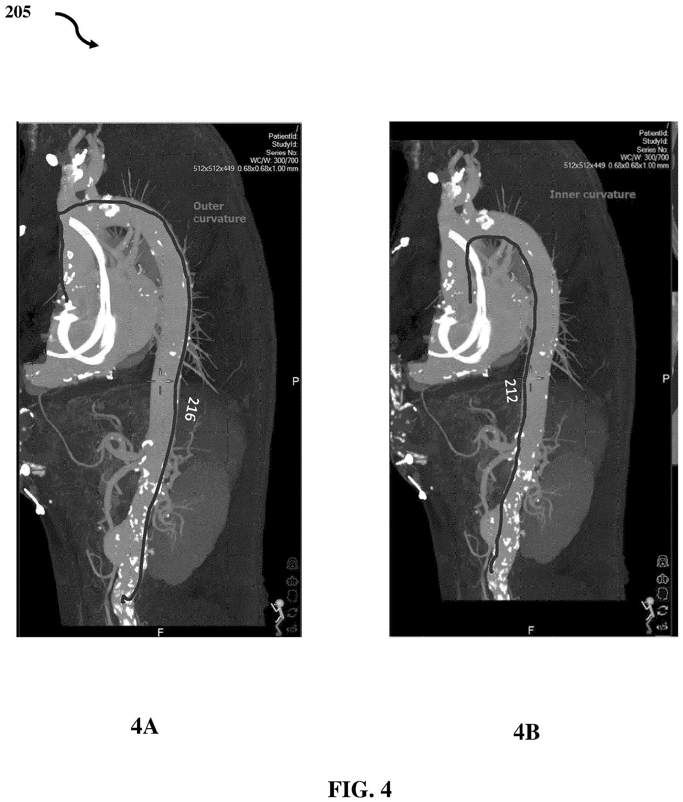

At step 106, the method 100 includes identifying the inner curve 212 and an outer curve 216 of the inner wall in the 3D segmentation 210 of the vessel 220 using a plurality of direction vectors 228 A-N orthogonal from the plurality of centerline points 222 A-N to the plurality of corresponding contour points 224 A-N. The plurality of direction vectors 228 A-N orthogonal from the plurality of centerline points 222 A-N to the plurality of corresponding contour points 224 A-N comprises the step of mapping the plurality of centerline points 222 A-N and the plurality of contour points 224 A-N into virtual latitudes 232 and longitudes 234. Fig. 4 is a representation of an identified inner curve 212 (as shown in 4B) and an outer curve 216 (as shown in 4A) according to an embodiment of the present invention.

The inner curve 212 and the outer curve 216 are recomputed from the modified centerline 214B and the modified contour (not shown), if the centerline underwent a modification, in other words, if an aneurysm was detected in the received data.

The method steps 104 and 106, along with the optimal approach for executing these steps, are elaborated upon in the following paragraphs.

In one embodiment of the present invention, when an aneurysm 236 is detected, the centerline 214A and contours 218 A-N in the affected region are recalculated using simple interpolation methods and resampled to maintain equidistant points. Additionally, smoothing and resampling techniques are applied along the longitudes 234 to prepare the data for distance calculations. This approach effectively reduces or mitigates the impact of outliers, such as noise, calcifications, localized compressions, or bulges, that need to be excluded from the tissue topology, thus enhancing the accuracy of geometric calculations along key longitudes 234 and latitudes 232. Distances along the longitudes 234 are computed using the Euclidean distance between consecutive points. Furthermore, tangent vectors at each centerline point 222 A-N are projected onto the orthogonal plane to generate direction vectors, which establish correspondence between the contour's longitudinal points.

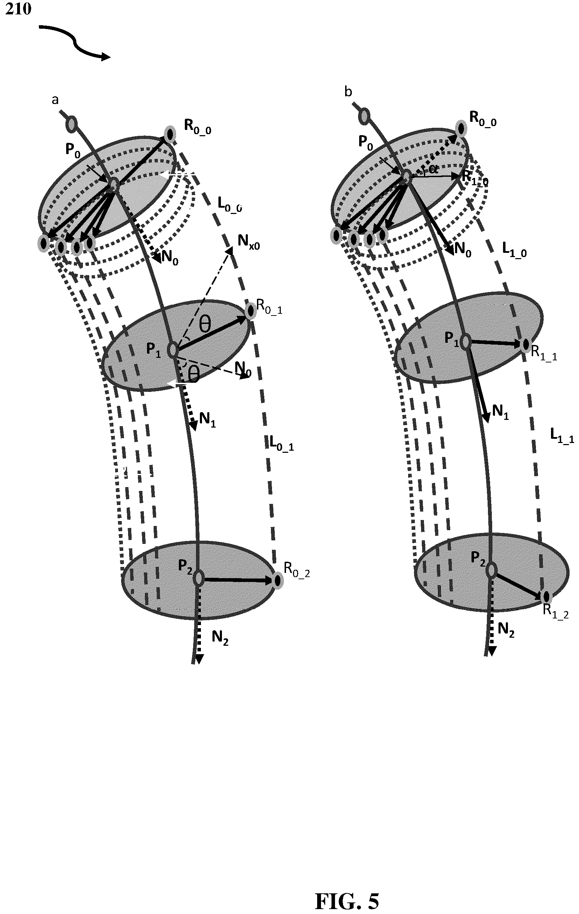

In accordance with such an embodiment, the proposed method (100) includes the said steps and can be understood by referring to the schematic of 3D segmentation 210 of the vessel 220 as illustrated in Fig. 5. It is to be noted, ribbons are shown with R₀ and R₁:
1. Initially, the method projects the 3D vector points of the contour and the raw 3D vector points of the centerline onto virtual latitudes and longitudes. The centerline points are aligned with the latitudes, while the contour points along the radius are mapped to the longitudes.
2. Next, ribbons are generated along the longitudes, with each latitude having a corresponding vector point. This is achieved by calculating a direction vector from the centerline point to the contour. The direction vectors are then rotated along the longitudes to create all the ribbons.
   To further elaborate on the methodology to establish correspondence between the contour longitudes to form the ribbons, the below steps are performed and can be understood with reference to Fig. 5.
   I. For the 0^{th} ribbon, find the vector tangential to the 0^{th} centerline point (Initial tangent), project this vector on to the orthogonal plane to get the first direction vector (initial direction vector) pointing towards the 0^{th} contour point. Repeat this process for all centerline points, to get a list of directions vectors along the latitudes at the 0^{th} angle.
   II. These direction vectors are multiplied by the maximum radius of the respective contours to get the contour pointers of the respective contours. The contour point closest to the contour pointer becomes a part of the 0^{th} ribbon, leading to the 0^{th} ribbon.
   III. Using the initial tangent and initial direction vector, rotate the direction vector at an angle to get the 0^{th} direction vector of the 1^{st} ribbon. Get the rest of the direction vectors along the latitudes (as mentioned in step 1), which forms the list of direction vectors of the 1^{st} ribbon. Get the 1^{st} ribbon following the method mentioned in step II.
   IV.Rotate the initial direction vectors at different angles (Recommended: (360 degree. /number of longitudes) *index of the longitude) to get a list of ribbons.
3. The ribbons are then smoothed using a weighted average technique, similar to Laplace smoothing, and resampled to ensure that the points are equidistant. The spacing between the points is made consistent with the centerline spacing.
4. The longest ribbon is identified and designated as the outer curve, while the shortest ribbon is defined as the inner curve.

In accordance with a further embodiment, particularly targeting an aneurysm, the proposed method involves the following steps:
1. The centerline in the region of the aneurysm is recalculated using Lagrange cubic interpolation and resampled to ensure equidistant points. The detailed steps are as follows:
   I. Lagrange cubic interpolation requires 4 points. These points are selected as follows: a point 10 positions before the start of the aneurysm [a1] (or the 0^{th} point of the centerline, if this point doesn't exist), the start point of the aneurysm [a2], the end point of the aneurysm [b1], and a point 10 positions after the aneurysm [b2] (or the last point of the centerline, if this point doesn't exist).
   II. The sampling boundaries between a2 and b1 are calculated, where omega is determined by the formula ((ala2 + blb2) / 2 * alb2), and 1 - omega gives the complementary boundary.
   III. The Lagrange polynomial is then sampled at different time intervals between these boundaries to generate new centerline points.
   IV.Finally, the points are resampled to ensure they are equidistant.
2. Next, the contours are recomputed based on the modified centerline in the aneurysm region:
   I. The average radii of the contours at the start and end points of the aneurysm are calculated. These values, along with the length of the modified centerline, are used to interpolate the radii of the contours between the two points using linear interpolation.
   II. Once the radii are established along the modified centerline latitudes, corresponding circular contours are calculated. These contours are derived by using a direction vector from the centerline point, with the radius length, and rotated along the longitude points.
3. The modified centerline and contours are then utilized, following the general steps outlined previously, to compute the inner and outer curves.

At step 108, the method 100 includes outputting (as shown in Fig. 4) the inner curve 212 and the outer curve 216 of the inner wall of the vessel 220 on a display together with the 3D medical image 205 and the 3D segmentation 210 of the vessel 220.Fig. 4 is a representation of an identified and displayed outer curve 216 (as shown in 4B) and an inner curve 212 (as shown in 4A) according to an embodiment of the present invention.

The method 100 of the invention offers significant advantages by delivering more accurate and robust results, while maintaining effectiveness even in scenarios with high levels of noise.

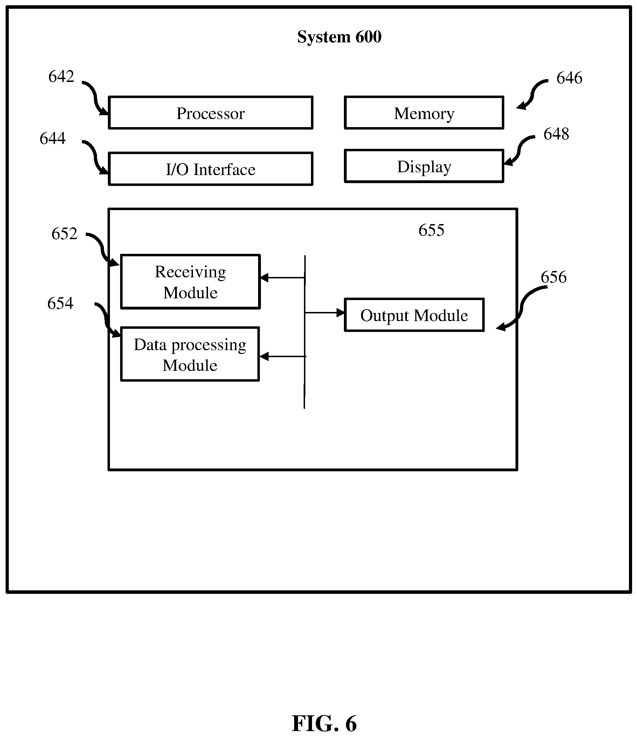

Fig 6 shows a block diagram of a system 600 for preprocedural planning, according to an embodiment of the present invention. The system 600 is configured to aid the clinician in preprocedural planning for endovascular stent-graft placement and/or computer assisted diagnosis.

Such a system 600 may be implemented in hardware or software or a combination thereof. The system 600 may include a processor 642, an Input/Output (I/O) interface 644, a memory 646, and a transceiver (not shown), a display 648 and a plurality of modules 655. In an exemplary embodiment, the processor 642 may be operatively coupled to each of the I/O interface 644, the plurality of modules 655, the transceiver and the memory 646.

The plurality of modules 655 may include but is not limited to, a receiving module 652, a data processing module 654 and a output module 656. The plurality of modules 655 may be implemented by way of suitable hardware and/or software applications. The processor 642 may include one or a plurality of processors. At this time, one or a plurality of processors may be a general-purpose processor, such as a central processing unit (CPU), an application processor (AP), or the like, a graphics-only processing unit such as a graphics processing unit (GPU), a visual processing unit (VPU), and/or an AI-dedicated processor such as a neural processing unit (NPU).

The system 600 may comprise the receiving module 652 for receiving image data from an imaging modality, for example, or from a database system. Such a database system may comprise a PACS server, for example. An image may comprise the 3D medical image 205. The 3D medical image 205 includes the 3D segmentation 210 of the vessel 220. In one example, the vessel 220 may be a blood vessel.

The receiving module 552 may be configured to receive the 3D medical image 205 comprising a 3D segmentation 210 of the vessel 220, wherein the 3D segmentation 210 comprises a centerline of the vessel 220 and a plurality of contours 218 A-N of an inner wall of the vessel 220. The centerline of the vessel 220 and the plurality of contours 218 A-N of the inner wall of the vessel 220 are represented as centerline points 222 A-N and contour points 224 A-N in a 3D space.

The data processing module 554 is configured to process the received 3D medical image 205 that includes the 3D segmentation 210 of the vessel 220 to further identify an inner curve 212 and an outer curve 216 of the inner wall of the vessel 220 in the 3D segmentation 210 of the vessel 220. The data processing module 554 is configured for using a plurality of direction vectors 228 A-N orthogonal from the plurality of centerline points 222 A-N to the plurality of corresponding contour points 224 A-N to identify an inner curve 212 and an outer curve 216 of the inner wall of the vessel 220. The plurality of direction vectors 228 A-N orthogonal from the plurality of centerline points 222 A-N to the plurality of corresponding contour points 224 A-N comprises the step of mapping the plurality of centerline points 222 A-N and the plurality of contour points 224 A-N into virtual latitudes 232 and longitudes 234.

The data processing module 554 is configured to identify a region of the vessel 220 in the 3D medical image 205 that comprises an aneurysm, recompute the centerline 214A in the identified region 236 to obtain a modified centerline 214B and generate the modified centerline 214B that replaces the centerline 214A.

In one embodiment, to recompute the centerline in the identified region 236, the one or more processors 542 are further configured to:
i. interpolate with a cubic polynomial using two points each on either side of the identified region 236, and
ii. resample the interpolated points to keep them equidistant, such as to identify a modified centerline in the identified region 236 and generate the modified centerline 214B that replaces the centerline 214A.

The one or more processors 642 are further configured to determine the plurality of contours 218 A-N between the contour points 224 A-N at the start 238A and at the end 238B of the identified region 236 in the 3D medical image 205, compute a modified radii of the plurality of contours 218 A-N in the identified region 236 of the 3D medical image 205 by interpolating a radii of the contour points 224 A-N of the plurality of contours 218 A-N along the entire length between the start 238A and the end 238B of the identified region 236 in the 3D medical image 205 and generate modified contours from the modified radii and the modified centerline 214B. The inner curve 212 and the outer curve 216 are computed from the modified centerline 214B and the modified contour.

The output module 556 is configured to output the inner curve 212 and the outer curve 216 of the inner wall of the vessel 220 on a display 548 together with the 3D medical image 205.

Advantageously, the system 500 of the invention can be used for evaluation of intuitive techniques for relatively more useful practical representation of segmentation of features derived from 3D contours, for example in vessel analysis.

Advantageously, the system 600 of the invention can be used for preprocedural planning in the placement of endovascular stent grafts for both elective and emergency scenarios involving thoracic and abdominal aortic aneurysms. The feature of the system 600 of the invention is to identify the inner curve 212 and the outer curve 216 of the inner wall of the vessel 220 using the 3D segmentation 210 which may assists clinicians in preprocedural planning for endovascular stent-graft placement by providing a more accurate estimation of the stent length, particularly in curved sections of the aorta. Thus, measuring both the inner and outer curve lengths of the vessel 220 using the embodiments of the invention more accurately reflects the deployed endograft's true length, surpassing the accuracy of centerline measurements in planning thoracic endografts. As the vessel's curvature and size increase, the underestimation caused by relying on centerline measurements becomes more pronounced. The approach of the system 600 for utilizing the outer curve and inner curve for the aorta shows promising outcomes, even when considering longitudinal strain and axial twists. Moreover, the accurate length measurement may also help to confirm suspected abnormalities. In addition to above, the longest and shortest longitudes across the lumen wall show promising results in proving to be the inner and outer curve of the lumen, in turn reasonably accurate length to support the stent length calculations.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile (transitory) and non-volatile (non- transitory) computer memory such as RAM, PROM, EPROM, and EEPROM. The storage media may be encoded with one or more programs that, when executed on one or more processors and/or controllers, perform at the required functions. Various storage media may be fixed within a processor or controller, may be transportable or may be available on-demand (e.g., via the cloud), such that the one or more programs stored thereon can be loaded into a processor or controller. While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

## Claims

1. A computer-implemented method (100) in particular for preprocedural planning and/or computer assisted diagnosis, the method (100) comprising the steps of:
- receiving (102), a 3D medical image (205) comprising a 3D segmentation (210) of a vessel (220), wherein the 3D segmentation (210) of the vessel (220) comprises a centerline (214A) of the vessel (220) and a plurality of contours (218 A-N) of an inner wall of the vessel (220), wherein the centerline (214A) of the vessel (220) and the plurality of contours (218 A-N) of the inner wall of the vessel (220) are represented as centerline points (222 A-N) and contour points (224 A-N) in a 3D space;
- identifying (106), an inner curve (212) and an outer curve (216) of the inner wall of the vessel (220) in the 3D segmentation (210) of the vessel (220) using a plurality of direction vectors (228 A-N) orthogonal from the plurality of centerline points (222 A-N) to the plurality of corresponding contour points (224 A-N); and
- outputting (108), the inner curve (212) and the outer curve (216) of the inner wall of the vessel (220) on a display together with the 3D medical image (205) and the 3D segmentation (210) of the vessel (220).

2. The computer-implemented method (100) according to claim 1, wherein using the plurality of direction vectors (228 A-N) orthogonal from the plurality of centerline points (222 A-N) to the plurality of corresponding contour points (224 A-N) comprises the step of mapping the plurality of centerline points (222 A-N) and the plurality of contour points (224 A-N) into virtual latitudes (232) and longitudes (234).

3. The computer-implemented method (100) according to claim 1, further comprising the steps of:
- identifying, a region (236) of the vessel (220) in the 3D medical image (205) that comprises an aneurysm (236);
- recomputing (104), the centerline (214A) in the identified region (236) for obtaining a modified centerline (214B); and
- generating the modified centerline (214B) that replaces the centerline (214A).

4. The computer-implemented method (100) according to claim 3, wherein the steps for recomputing the centerline (214A) in the identified region (236) comprise (i) interpolating with a polynomial using two points each on either side of the identified region (236) and (ii) resampling the interpolated points to keep them equidistant, such as to identify the modified centerline (214B) in the identified region (236).

5. The computer-implemented method (100) according to claim 4, further comprising the steps of:
- determining the plurality of contours (218 A-N) between the contour points (224 A-N) at the start (238A) and at the end (238B) of the identified region (236) in the 3D medical image (205);
- computing a modified radii of the plurality of contours (218 A-N) in the identified region (236) of the 3D medical image (205) by interpolating a radii of the contour points (224 A-N) of the plurality of contours (218 A-N) along the entire length between the start (238A) and the end (238B) of the identified region (236) in the 3D medical image (205); and
- generating modified contours from the modified radii and the modified centerline (214B).

6. The computer-implemented method (100) according to claim 5, wherein the inner curve (212) and the outer curve (216) are computed from the modified centerline (214B) and the modified contour.

7. The computer-implemented method (100) according to claim 6, using the computed inner curve (212) and the outer curve (216) for estimating a stent length for preprocedural planning and/or computer assisted diagnosis.

8. A system (600) in particular for preprocedural planning and/or computer assisted diagnosis, the system (600) comprising:
a display (648); and
one or more processors (642) configured to:
- receive a 3D medical image (205) comprising a 3D segmentation (210) of a vessel (220), wherein the 3D segmentation (210) of the vessel (220) comprises a centerline (214A) of the vessel (220) and a plurality of contours (218 A-N) of an inner wall of the vessel (220), wherein the centerline (214A) of the vessel (220) and the plurality of contours (218 A-N) of the inner wall of the vessel (220) are represented as centerline points (222 A-N) and contour points (224 A-N) in a 3D space;
- identify an inner curve (212) and an outer curve (216) of the inner wall of the vessel (220) in the 3D segmentation (210) of the vessel (220) using a plurality of direction vectors (228 A-N) orthogonal from the plurality of centerline points (222 A-N) to the plurality of corresponding contour points (224 A-N); and
- output the inner curve (212) and the outer curve (216) of the inner wall of the vessel (220) on a display together with the 3D medical image (205) and the 3D segmentation (210) of the vessel (220).

9. The system (600) according to claim 8, wherein using the plurality of direction vectors (228 A-N) orthogonal from the plurality of centerline points (222 A-N) to the plurality of corresponding contour points (224 A-N) comprises the step of mapping the plurality of centerline points (222 A-N) and the plurality of contour points (224 A-N) into virtual latitudes (232) and longitudes (234).

10. The system (600) according to claim 8, wherein the one or more processors (642) are further configured to:
- identify a region of the vessel (220) in the 3D medical image (205) that comprises an aneurysm;
- recompute the centerline (214A) in the identified region (236) to obtain a modified centerline (214B); and
- generate the modified centerline (214B) that replaces the centerline (214A).

11. The system (600) according to claim 10, wherein to recompute the centerline in the identified region (236), the one or more processors (642) are further configured to (i) interpolate with a polynomial using two points each on either side of the identified region (236) and (ii) resample the interpolated points to keep them equidistant, such as to identify the modified centerline (214B) in the identified region (236).

12. The system (600) according to claim 11, wherein the one or more processors (642) are further configured to:
- determine the plurality of contours (218 A-N) between the contour points (224 A-N) at the start (238A) and at the end (238B) of the identified region (236) in the 3D medical image (205);
- compute a modified radii of the plurality of contours (218 A-N) in the identified region (236) of the 3D medical image (205) by interpolating a radii of the contour points (224 A-N) of the plurality of contours (218 A-N) along the entire length between the start (238A) and the end (238B) of the identified region (236) in the 3D medical image (205); and
- generate modified contours from the modified radii and the modified centerline (214B).

13. The system (600) according to claim 12, wherein the inner curve (212) and the outer curve (216) are computed from the modified centerline (214B) and the modified contour.

14. The system (600) according to claim 13, wherein the one or more processors (642) are further configured to estimate a stent length for preprocedural planning and/or computer assisted diagnosis by using the computed inner curve (212) and the outer curve (216).

15. A computer program comprising instructions which, when the program is executed by a computer, cause the computer to carry out the method according to any one of claim 1 to 7.
